# EUROPEAN PATENT SPECIFICATION

(11) **EP 2 407 128 B1**
(45) Date of publication and mention of the grant of the patent: **25.03.2015**
(21) Application number: 11173486.9
(22) Date of filing: 11.07.2011
(51) Int. Cl.: A61F 5/01

(54) **JOINT BRACE**
ORTHESENGELENK
SUPPORT D'ARTICULATION

(30) Priority: 12.07.2010 IT TV20100098
(43) Date of publication of application: 18.01.2012
(73) Proprietor: Giani, Marco, 31044 Montebelluna (TV) (IT)
(72) Inventor: Giani, Marco, 31044 Montebelluna (TV) (IT)
(74) Representative: Citron, Massimiliano

(56) References cited:
- EP-A1- 0 820 741
- WO-A1-2007/006358
- DE-C- 663 643
- US-A- 6 080 121
- US-A1- 2002 148 461

## Description

Articulated braces are known with the function to bandage/wrap and support a joint. As an example we discuss here knee braces. The braces of this type fall into two categories: wrapping braces and articulated braces (e.g. see WO 2009061376 or U.S. 2004097859).

The first are essentially an elastic band that can be tightened around the knee with pull-fasteners, usually Velcro. Often they also have a patellar hole to facilitate the leg flexion. The second have instead an open rigid structure which is jointed at the knee. By means of buckles or Velcro strips one can fix the structure to the thigh and the calf. Both models have several disadvantages.

The wrapping braces are all closed, and wrap the leg completely. Their adjustment to fit them to the leg is poor, because it is only possible at the end of the brace and not e.g. close to the patella. Very often to apply the brace firmly one has to exaggerate the fastening grip, with little comfort for the user. In addition, pull-fasteners when tightened on the edges of the brace tend to make the brace edges lift, reducing the grip capacity on the leg.

Even the articulated braces give little freedom of adjustment. They can be adjusted only on the - limited - anchor areas. The underlying muscle is stressed only on a small area, with possible blood circulatory problems. Moreover, their rigid structure makes them cumbersome and not at all packable.

WO 2007006358 describes a knee brace having tightening laces for tying two bands above and below the articulation. The laces act as multiple pull-straps attached to a handle and have anchorage fixed points to the body of the brace. The lace system is awkward to tighten, requires a lot of strength, and the knee joint is uncovered and not supported, since there are only two articulated rods on its sides.

US 6 080 121 discloses an ankle brace with multiple straps includes an outer mesh surface that surrounds the patient's foot and a plurality of eyelets that are used to lace-up the front of ankle brace.

A knee brace as defined in the preamble of claim 1 is disclosed in DE-C-663 643.

The main object of the invention is to improve this state of the art.

Other objects are to (i) solve the problem of a corrected and improved attachment of the brace to the user body, without the aforementioned disadvantages, and/or (ii) provide a brace with very precise adjustment.

Such objects are achieved by a brace as in Claim 1.

The presence of laces ensures accurate, uniform and easy adjustment of the brace around the articulation and the attached limbs. This prevents lifting of the edges as in Velcro fasteners and their intrinsic point-like traction.

As preferred variants the brace comprises interruption or slackening (free-ended) hems of the tubular structure that extend substantially parallel to each other and parallel to the plane in which the articulated limbs move (in the case of the bent leg it is e.g. the plane containing the femur and tibia), the one or more laces are configured for bringing nearer or taking in said hems one toward the other.

Preferably the hems to be tightened extend over all the length of the brace, so the brace when not in use is a flat body that is comfortably worn by wrapping it around the articulation and then fixing it to the articulation by tightening the laces that tighten the hems.

The coupling of the laces to the flexible body or sheath can be made through their insertion in pockets obtained on the body or sheath, or inside close, hollow support or channels, e.g. made from plastic material or leather, sewn to the body or sheath. Other preferred variants are defined in the dependent claims.

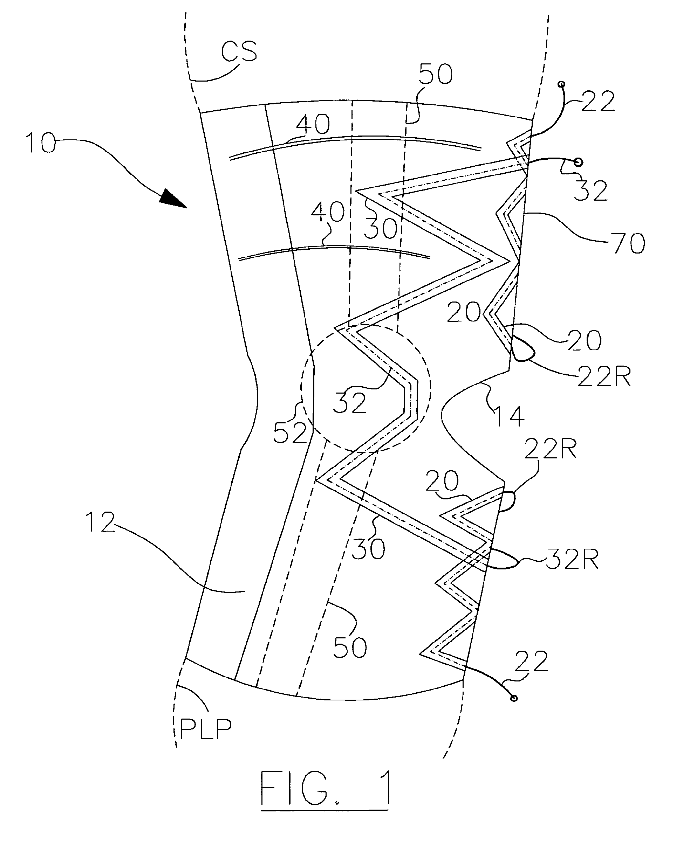

The invention and its advantages will be more clear from the following description of a preferred embodiment, illustrated in the drawings in which
Figure 1 shows a side view of a brace;
Figure 2 shows a front view of the brace of Figure 1.

Shown in the figures is a joint brace 10 according to the invention. It is formed by a body or sleeve 12 that is flexible. The body 12 has two hems 70 and can be bent by bringing the hems 70 nearer to form a tubular bandage with which to wrap the leg. Nearly at the middle of each hem 70 is an inlet 14 that allows the knee (patella) to be received therein when the body 12 in use is wrapped around the leg to form a sleeve (as in the figures). The ends of the limbs forming the joint (in the example the thigh CS and calf PLP) become wrapped and bind by the distal parts of the body 12.

Coupled to the hems 70 are two laces 22, for example by through-holes or slots made in the body 12, rings or fins attached to the body 12 or within channels 20 made in the body 12, e.g. by passing a lace 22 between two sewn layers or tapes. As already mentioned the channels 20 can also be tubular elements or strips, e.g. made of plastic material or leather, applied by stitching, heat-sealing, or other technology to the body 12. The advantage is the increased strength of the brace.

In particular, from one side and the other with respect to the inlet 14 (corresponding to the area over the thigh CS and over the calf PLP) there is a lace 22 that connects at least one point of opposing sides of the hems 70. The path of the laces 22 can be rectilinear, i.e. substantially parallel to the edge of the hems 70 to better cover and tighten the hems 70, or with zig-zag pattern along a feed direction substantially parallel to the edge of the hems 70, so as to involve a greater area of the hems 70.

As stated, each lace 22 is hooked at least once to each hem 70. Ways and configurations to do this are e.g. all known lacing methods for shoelaces. The function of the lace 22 and/or a plurality of laces 22 arranged as described is to allow the user to pull the hems 70 toward one another, thereby tightening the brace 10 and securing it firmly to the leg. To limit the number of laces 22 a loop of lace can be created, with a bridge lace 22R that passes from a hem 70 to the other. Thus the two ends of a lace 22 are on the same side and can be easily pulled. As convenient variants the hems 70 can be laced by a single lace, or one can use a lace for the portion of hem 70 that is on one side of the inlet 14 (therefore two laces all in all as in the figures) or one can divide the length of the laces 70 and make each of these facing hem fractions to be laced and tightened with an independent lace. Or the brace 10 can foresee in the area of the knee joint a lateral and medial connection system between the upper part (thigh CS) and lower part (calf PLP) with support strips, preferably rigid, in which the laces 22 run or to which the laces 22 are coupled.

On each hem 70 there is a lace 32 coupled to the body 12, e.g. by using the same means 30 of the laces 22. The lace 32 is arranged so as to surround the opening/inlet 14 and extend for some distance in a direction parallel to the hems 70. It is convenient to use only a single lace 32, which runs on the two hems 70 and almost completely surrounds the inlet 14 passing from a hem 70 to the other and reversing its length direction by 180° at a segment 32R that acts as bridge. Preferably the lace 32 is placed on the body 12 with a zig-zag path in order to achieve the structural and tensioning effects described for the lace 22. In this case, what has been said for the variants of the laces 22 holds as well. The laces 32 allow for adjustment both above and below to the patella area, in order to control the so-called anterior drawer.

Approximately perpendicular to the hems 70 are arranged in the body 12 support or stiffening strips 40, arranged e.g. into pockets sewn directly into or onto the body 12.

Optionally in the body 12 there can be present the lateral rigid rods 50, e.g. fixed in pockets sewn into it, which converge (extending approximately parallel to the hems 70) in the patellar or articulation zone to a polycentric hinge 52. The rigid rods 50 are coupled to the body 12 so that when it is applied to the leg, they are located on either side of the articulation. The rigid rods 50, made e.g. from aluminum or plastic or composite fibers, along with the polycentric hinge allow to use the knee articulation in agonistic scenarios as well.

The brace 10 is used as follows.

Wrapped the body 12 around the leg, the patella is made to coincide with the opening formed by the inlets 14 adjacently located. Then the laces 22 are pulled to bring the hems 70 closer and tighten the body 12 to the leg. Note that in this phase the brace 10 allows the total and uniform wrapping of the thigh CS and calf PLP thanks to the lace closing system, which also allows for micrometric adjustment. The big problem of blocking effect, in areas of the quadriceps and calf, for muscle bundles, which occurs in the known knee-braces, is eliminated.

Then the laces 32 are pulled to stiffen the lateral areas of the knee. The lace system made by the lace 32 has the goal to protect and prevent possible injuries to the anterior collateral ligament caused by abnormal movements of the knee, especially during action-sport practice (skiing, tennis, football, basketball, etc...).

The body 12 is e.g. made of latex or lycra or cordura. Generally it is convenient that e.g. when using a rigid elasticized material for the body 12 a material, which is extensible as well as e.g. rigid, is used for the coupling means 20, 30 of laces 22, 32. When for the body 12 an elastic material is used, for the coupling means 20, 30 it is preferable to use inextensible material and/or reinforcements, e.g. even rigid.

Preferably the body 12 is designed so that the hems 22 remain parallel to each other along the whole articulation, and preferably at a distance from one another and parallel to the plane wherein the two articulated limbs move. In this way a central strip of the leg is not covered by the material of the brace, but only by the laces 22. This improves circulation and perspiration. It is possible to create openings or windows in the body 12 for ventilation and/or lightening, however always without affecting the structural strength.

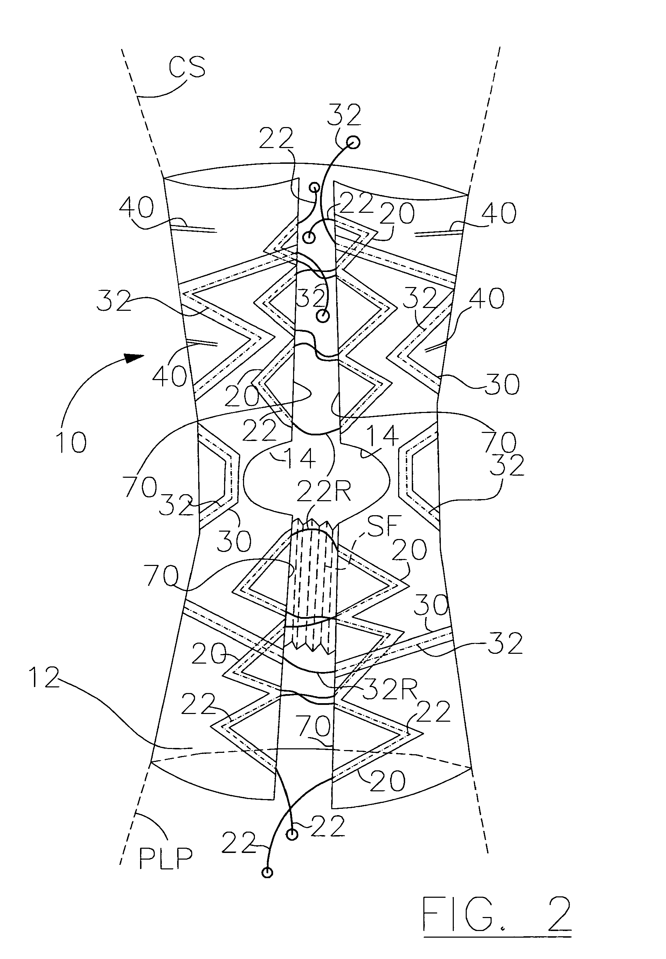

Note however that the hems 70 need not necessarily cross the entire brace 10 and thus the articulation, but can e.g. interrupt at one or more conjunction and continuity bands or areas SF between one side of the articulation and the other, where the tubular structure of the brace 10 is continuous and without interruptions over 360° (see strip SF hatched in Figure 2). These areas SF, where the brace is continuous and/or all closed (i.e. it is locally perfectly tubular), can include ribbons or strips of protective and/or elastic and/or extensible material, e.g. with bellow structure preferably elasticized, so as to improve the adaptability of the brace 10 on limbs. Above the areas SF, a lace 22 can in any case extend to shorten and/or tighten the section of the brace 10 in that area, or such areas can be without laces and adapt to the limb below, either due to their own elasticity or simply by overlapping.

## Claims

1. Knee brace (10) adapted to wrap the knee articulation and support the movement thereof, comprising
- a flexible body or sheath (12) adapted to form or configure itself in use like a tubular structure for wrapping substantially all the articulation and the ends of the thigh and calf (CS, PLP), and
- one or more laces (22, 32) that are slidingly coupled on said body in traction points or zones, e.g. eyelet formations or channels, and are pullable for reducing the section of the tubular structure and/or taking in and/or adapting said body (12) on the articulation
**characterized in that**
it comprises one or more laces (32) coupled to the zone (14) of the flexible body that - in use - is placed all around the patella, said one or more laces (32) being adapted for taking in and/or tensioning said zone to put it in traction.

2. Brace according to claim 1, comprising interruption or slackening hems (70) of the tubular structure that extend substantially parallel to each other and parallel to the plane in which the articulated limbs move, the one or more laces (22, 32) being configured for bringing nearer or taking in said hems one toward the other.

3. Brace according to claim 2, wherein said hems (70) extend over all the length of the brace (12).

4. Brace according to one of the preceding claims 1 to 3, comprising a single lace (32) coupled to said body all around the zone which - in use - is placed all around the articulation joint, said single lace (32) being adapted for taking in and/or tensioning said zone to put it in traction.

5. Brace according to claims 2 and 4, wherein said single lace (32) is coupled with, and runs in direction substantially parallel to, hems that are opposites to each other and arranged - in use - all around the articulation joint, said single lace (32) comprising a segment (32R) that passes from a hem to the opposite one for a change of direction.

6. Brace according to any one of the previous claims, wherein a or each lace extends on the surface of and/or inside said body (12) and/or on the surface of and/or inside said hems with a zig-zag pattern.

7. Brace to any of the previous claims, wherein a or each lace extends on the surface of or inside said body (12) with a substantially looped or U-pattem, such that its extremities are near each other and pullable by equal verse.

8. Brace according to any one of the preceding claims 2 to 7, wherein said body (12) is made of inextensible material and the brace comprises elongated reinforcing elements which are elastically deformable and coupled to or integrated in said body and arranged perpendicularly to said hems, or
wherein said body (12) is elastic and the brace comprises elongated reinforcing elements (40) which are rigidly deformable and coupled to or integrated in said body and arranged perpendicularly to said hems.

9. Brace according to any one of the previous claims, comprising two pairs of rigid rods (50) that are connected together by a joint, and coupled to or integrated into said body (12) so as to be placed - with the brace in use - at the articulation sides to support it.

10. Brace according to any one of the previous claims, wherein one or more laces are slidingly coupled on said body by closed channels that are coupled to said body.

## Patentansprüche

1. Kniestütze (10) welche die Aufgabe hat, das Kniegelenk zu umschließen und die Bewegung desselben zu unterstützen, bestehend aus
- einem flexiblen Körper oder Hülle (12), welche während der Anwendung eine röhrenförmige Struktur bildet und sowohl das gesamte Gelenk als auch die Ausläufer des Oberschenkels und der Wade (CS, PLP) umschließt, und
- einem oder mehr Bändern (22, 32), welche in bestimmten Zugpunkten oder Zonen auf dem zuvor genannten Körper angebracht sind und durch z.B. Zugössen oder Kanäle gleiten; die Bänder können gezogen werden, um den Abschnitt der röhrenförmigen Struktur verkleinern zu können und/oder somit den Körper (12) an das Kniegelenk genau anpassen zu können,
**gekennzeichnet dadurch**, dass
ein oder mehrere Bänder (32) vorhanden sind, welche in der Zone (14) des flexiblen Körper verschlungen werden; während der Anwendung, wird mittels einem oder mehreren Bändern (32), die Struktur der Kniescheibe angepasst.

2. Stütze, welche der Anforderung 1 entspricht, mit Unterbrechungen oder Säumen (70) zum Nachlassen der Spannung der röhrenförmigen Struktur, parallel zueinander angeordnet und parallel zur Ebene, in welcher sich die Gelenke bewegen; ein oder mehrere Bänder (22, 32) ziehen die Stütze in diesen Säumen zusammen oder werden darin verschlungen.

3. Stütze, welche der Anforderung 2 entspricht, und die Säume (70) sich über die gesamte Länge der Stütze (12) erstrecken.

4. Stütze, welche einer der Anforderungen 1-3 entspricht, ausgestattet mit einem einzigen Band (32), welches - bei Anwendung - den Körper um das gesamte Gelenk festigt; das Band (32) passt die Struktur an und/oder bringt die gesamte Zone in Traktion.

5. Stütze, welche den Anforderungen 2 und 4 entspricht, wobei ein einziges Band (32) mit Säumen verbunden ist, und parallel zu diesen verläuft; die Säume sind gegenüber liegend angeordnet und erstrecken sich über das gesamte Gelenk; zum Richtungswechsle des Bandes (32) ist ein Segment (32R), welches von einer Saumseite zur anderen verläuft, angebracht.

6. Stütze, welche einer der zuvor genannten Anforderungen entspricht, wobei eines oder jedes Band sich an der Oberfläche und/oder im Körper (12) und/oder an der Oberfläche und/oder im Inneren der Säume zig-zag bewegt.

7. Stütze, welche einer der zuvor genannten Anforderungen entspricht, wobei eines oder jedes Band sich an der Oberfläche oder im Körper (12) U-förmig bewegt, sodass sich die Enden der Bänder gegenüber liegend befinden und in dieselbe Richtung gezogen werden können.

8. Stütze, welche einer der zuvor genannten Anforderungen von 2 bis 7 entspricht, wobei der Körper (12) aus dehnbarem Material hergestellt ist und die Stütze längliche Verstärkungselemente, welche elastisch verformbar sind und mit dem Körper verbunden und/oder in diesem integriert sind, enthält und senkrecht zu den Säumen angeordnet sind, oder
Wobei der Körper (12) elastisch ist die Stütze längliche Verstärkungselemente (40) beinhaltet, welche steif verformbar sind und mit dem Körper verbunden und/oder in diesem integriert sind, enthält und senkrecht zu den Säumen angeordnet sind.

9. Stütze, welche einer der zuvor genannten Anforderungen entspricht, wobei zwei Paar starre Stäbe (50) durch eine Gelenkverbindung gekoppelt werden, und mit dem Körper (12) verbunden oder in diesen integriert sind, sodass diese das Gelenk - wenn die Stütze verwendet wird - unterstützen.

10. Stütze, welche einer der zuvor genannten Anforderungen entspricht, wobei eines oder mehrere gleitende Bänder mit dem Körper mittels geschlossenen Kanälen, welche am Körper befestigt sind, in Verbindung stehen.

## Revendications

1. Attelle de genou (10) adaptée pour être enveloppée autour de l'articulation genou et soutenir le mouvement de celui-ci, comprenant :
• Un corps flexible ou gaine (12) adapté pour former ou se configurer de manière à pouvoir être utilisée comme structure tubulaire enveloppant pratiquement toute l'articulation et les extrémités de la cuisse et du mollet (CS, PLP) et
• Une ou plusieurs attaches (22, 32) qui sont reliées en coulissant sur ce corps à des points ou zones de traction, par ex. oeillets ou canaux, et celles-ci peuvent être tirées pour réduire le diamètre de la structure tubulaire et/ou serrer et/ou être adaptées au corps (12) au niveau de l'articulation.
cette attelle est **caractérisée par** :
le fait qu'elle comprend une ou plusieurs attaches (32) reliées à la zone (1) du corps flexible qui, quand cette attelle est utilisée, est placé tout autour de la rotule ; ces attaches (32) sont alors adaptées pour serrer et/ou tendre cette zone afin de la mettre en traction.

2. Attelle, selon la revendication 1, comprenant une interruption ou des bords retournés de relâchement (70) de la structure tubulaire qui s'étirent pratiquement parallèlement l'un par rapport à l'autre et parallèlement à la direction dans laquelle les membres articulés bougent, l'attache ou les attaches (22, 32) étant configurées pour rapprocher ou serrer ces rebords l'un vers l'autre.

3. Attelle, selon la revendication 2, sur laquelle ces rebords (70) s'étirent au-dessus de la longueur totale de l'attelle (12).

4. Attelle, selon une des revendications précédentes 1 à 3, comprenant une seule attache (32) reliée au corps, tout autour de la zone, quand l'attelle est utilisée ; cette attache unique (32) est placée tout autour de la rotule et est adaptée pour serrer et/ou tendre la zone à mettre en traction.

5. Attelle, selon les revendications 2 et 4, où l'attache unique (32) est reliée à des rebords et longe parallèlement ceux-ci, qui sont opposés l'un à l'autre et sont fixés, pendant l'utilisation de cette attelle, tout autour de la rotule ; cette attache simple (32) comprenant un segment (32R) passe d'un rebord à celui opposé pour changer de direction.

6. Attelle, selon une des revendications précédentes quelconque, où une seule ou chaque attache s'étire sur la surface et/ou à l'intérieur du corps (12), et/ou sur la surface et/ou à l'intérieur des rebords avec une configuration en zigzag.

7. Attelle, selon une des revendications précédentes quelconque, où une seule ou chaque attache s'étire sur la surface ou à l'intérieur du corps (12) avec une configuration pratiquement en boucle ou en U, de façon à ce que ses extrémités soient proches l'une de l'autre et puissent être tirées du même côté.

8. Attelle, où, selon une des revendications précédentes quelconque de 2 à 7, le corps (12) est réalisé dans un matériau non étirable et l'attelle comprend des éléments de renforcement étirés qui peuvent être déformés du point de vue élastique et être reliés ou intégrés au corps et placés perpendiculairement aux rebords ou
Où le corps (12) est élastique et l'attelle comprend des éléments renforcés étirés (40) qui sont déformables mais rigides et sont reliés ou intégrés au corps et placés perpendiculairement aux rebords.

9. Attelle, selon une des revendications précédentes quelconque, comprenant deux paires de cordons rigides (50) qui sont reliés entre eux par un joint et reliés ou intégrés au corps (12) de manière à être placés, quand l'attelle est utilisée, sur les côtés de l'articulation pour la soutenir.

10. Attelle, selon une des revendications précédentes quelconque, où une ou plusieurs attaches sont reliées par coulissement sur le corps, par des canaux fermés qui sont fixés au corps.
